# DEMANDE DE BREVET EUROPEEN

(11) **EP 0 582 533 A1**
(43) Date de publication de la demande: **09.02.1994**
(21) Numéro de dépôt: 93440061.5
(22) Date de dépôt: 05.08.1993
(51) Int. Cl.: A61F 9/00, A61B 17/32

(54) **Instrument chirurgical pour la fragmentation in situ d'un matériau vivant**

(30) Priorité: 06.08.1992 FR 9209970
(71) Demandeur: LABORATOIRES DOMILENS SOCIETE ANONYME, F-69007 Lyon (FR)
(72) Inventeur: Charleux, Jacques, F-69001 Lyon (FR)

(57) **Abrégé**

Instrument chirurgical pour la fragmentation in situ d'un matériau vivant dans un organe (1) ou une partie d'un corps humain ou animal, et l'extraction dudit matériau dudit organe, ledit instrument comprenant, d'une part un outil de fragmentation (2), et d'autre part un moyen d'actionnement (5) de l'outil au contact direct du matériau vivant, à fragmenter et extraire, caractérisé en ce que l'outil est un outil de travail à sec du matériau vivant et comprend à l'intérieur d'un manchon un organe rotatif (6) de découpe et évacuation longitudinale du matériau vivant, associant, d'une part au moins un moyen rotatif de coupe (7a,81,82), susceptible de découper le matériau vivant au travers d'une fenêtre (3c), et d'autre part une vis d'évacuation (7) du matériau vivant découpé, vers l'extrémité distale (3a) de l'outil (2). Un moyen d'actionnement (5) de l'outil comprend un moyen mécanique (9) d'entraînement en rotation de l'organe rotatif (6) de découpe et évacuation longitudinale du matériau vivant.

## Description

La présente invention a trait à un instrument chirurgical notamment de microchirurgie, permettant à la fois de fragmenter in situ un matériau vivant ayant une certaine consistance, dans un organe ou une partie d'un corps humain ou animal, et d'extraire ledit matériau ainsi fragmenté.

Quoique non limitée à cette application, la présente invention sera introduite, définie et décrite par référence à la phaco-fragmentation ou vitrectomie de l'oeil. Cette technique chirurgicale permet en effet de fragmenter in situ le noyau du cristallin, relativement dur, par exemple en cas de cataracte, et d'extraire de l'oeil le cristallin ainsi fragmenté. Postérieurement à ce travail, un implant ou cristallin artificiel est ensuite mis en place dans le sac capsulaire, en lieu et place du cristallin extrait.

Jusqu'à présent, tout instrument de phaco-fragmentation travaille en utilisant l'energie mécanique et vibratoire des ultra-sons, et par la voie humide. En effet, classiquement un instrument de phaco-fragmentation comprend un outil de fragmentation ou émulsification, et un moyen d'actionnement dudit outil, par exemple à ultra-sons ou magnéto-strictif.

L'outil proprement dit comporte un manchon rigide allongé, et plus précisément un aiguille métallique creuse, d'insertion dans l'oeil, et plus précisément à travers la cornée dans les chambres antérieure et postérieure de l'oeil. Cette aiguille s'étend d'une extrémité distale destinée à rester l'extérieure de l'oeil, à une extrémité proximale destinée à venir à l'intérieur de l'oeil, en relation et au contact du noyau du cristallin. Un tube extérieur entoure à distance le manchon, en laissant libre son extrémité proximale, et en ménageant avec ledit manchon un canal annulaire. Le tube extérieur comporte une ouverture d'introduction par le canal annulaire d'un liquide de substitution de l'humeur aqueuse de l'oeil, tandis que l'aiguille comporte une fenêtre antérieure, située au moins pour partie dans la paroi latérale, du côté de son extrémité proximale, mettant en communication l'intérieur avec l'extérieur de ladite aiguille, pour l'extraction du liquide de substitution entraînant les fragments du cristallin.

Le moyen d'actionnement permet de mouvoir l'outil, longitudinalement et alternativement dans un sens et dans l'autre, au contact direct du matériau vivant à fragmenter et extraire. Ce moyen consiste en une tête équipée d'un générateur à ultra-sons ou magnéto-strictif, sur laquelle l'extrémité distale de l'outil de fragmentation peut être montée de manière amovible, par exemple par vissage.

En pratique, si l'on veut une fragmentation effectuée avec précision à l'endroit du noyau dur du cristallin, il est nécessaire d'introduire une spatule de maintien ou écartement du cristallin, par une autre incision sur la cornée. Cette spatule permet en effet de positionner le cristallin, de manière indépendante des flux liquides en circulation, là où l'outil de fragmentation peut agir.

Après fragmentation, un tel instrument procède donc par aspiration, au travers de la fenêtre antérieure de l'aiguille creuse, de l'humeur aqueuse et du liquide de substitution apporté par le tube extérieur, le tout entraînant les fragments du cristallin détruit.

Une telle aspiration apporte avec elle un certain nombre d'inconvénients.

Pour respecter la pression oculaire, et dans la chambre antérieure et dans la chambre postérieure de l'oeil traité, il convient non seulement d'amener dans ce dernier un liquide de substitution de l'humeur aqueuse, mais aussi de contrôler soigneusement et d'équilibrer les pressions d'infusion et d'extraction des liquides en circulation.

De manière incontrôlée, ne serait-ce qu'en raison des fragments susceptibles d'obstruer la fenêtre antérieure, des à-coups d'aspiration peuvent se produire à l'extrémité proximale de l'outil de fragmentation, attirant la paroi capsulaire à proximité, voire au contact de l'outil de fragmentation, avec toutes les conséquences opératoires dommageables qui peuvent en résulter pour ladite paroi.

La présente invention a donc pour objet un instrument chirurgical de fragmentation et évacuation, notamment de phaco-fragmentation, obviant les inconvénients liés à l'aspiration d'un liquide.

Selon la présente invention, l'outil de fragmentation est un outil de travail à sec du matériau vivant. Il comprend, à l'intérieur du manchon d'insertion dans l'organe traité, un organe rotatif de découpe et évacuation longitudinale du matériau vivant, associant, d'une part au moins un moyen rotatif de coupe, en vis-à-vis de la fenêtre antérieure du manchon, susceptible de découper le matériau vivant au travers de ladite fenêtre, et d'autre part une vis d'évacuation du matériau vivant découpé, vers l'extrémité distale de l'outil. En correspondance, le moyen d'actionnement de l'outil comprend un moyen mécanique d'entraînement en rotation de l'organe rotatif de découpe et d'évacuation longitudinale.

S'agissant des instruments de phaco-fragmentation, un instrument selon l'invention rompt donc complètement, et avec la technique des ultra-sons, et avec la technique de circulation de liquides au sein de l'oeil traité.

En recourant à une action purement mécanique, à savoir un véritable usinage tangentiel du cristallin, la solution selon l'invention permet de s'affranchir des limites des techniques antérieures.

Consécutivement, on obtient les avantages essentiels suivants :
1) puisque procédant mécaniquement à un véritable usinage du cristallin ou de son noyau, la fragmentation devient indépendante de la dureté ou consistance dudit noyau; en particulier, avec un instrument selon l'invention, on peut attaquer des noyaux particulièrement durs, jusqu'ici réfractaires ou difficiles à traiter ou travailler avec les instruments usuels à ultra-sons ou magnéto-strictifs
2) puisque supprimant toute infusion/aspiration dans l'oeil, on évite toute perturbation hydrostatique interne, telle que résultant antérieurement du renouvellement de l'humeur aqueuse, allant par exemple jusqu'à 200 fois le volume interne de l'oeil
3) l'instrument selon l'invention, libéré de toute circulation annexe de liquide, apporte au chirurgien une bien meilleure sensitivité de son geste opératoire.

La présente invention est maintenant décrite par référence aux dessins annexés, dans lesquels :
- la figure 1 représente, de manière schématique et en coupe axiale, un instrument chirurgical, et plus précisément de phaco-fragmentation selon l'invention
- la figure 2 représente le mode de raccordement d'un outil de fragmentation et d'un moyen d'actionnement, appartenant à un instrument chirurgical selon la figure 1
- la figure 3 représente une autre forme de réalisation d'un outil de fragmentation selon l'invention
- la figure 4 représente une vue en coupe selon la ligne IV-IV de l'outil de coupe représenté à la figure 3
- la figure 5 représente une vue en coupe d'un outil de fragmentation selon l'invention, mais réalisé selon une autre modalité de construction selon laquelle les fonctions de coupe et évacuation longitudinale sont séparées
- la figure 6 représente, vu en coupe et de manière schématique, l'instrument chirurgical intégrant l'outil de fragmentation selon la figure 5
- la figure 7 représente le mode d'utilisation d'un instrument chirurgical selon l'invention.

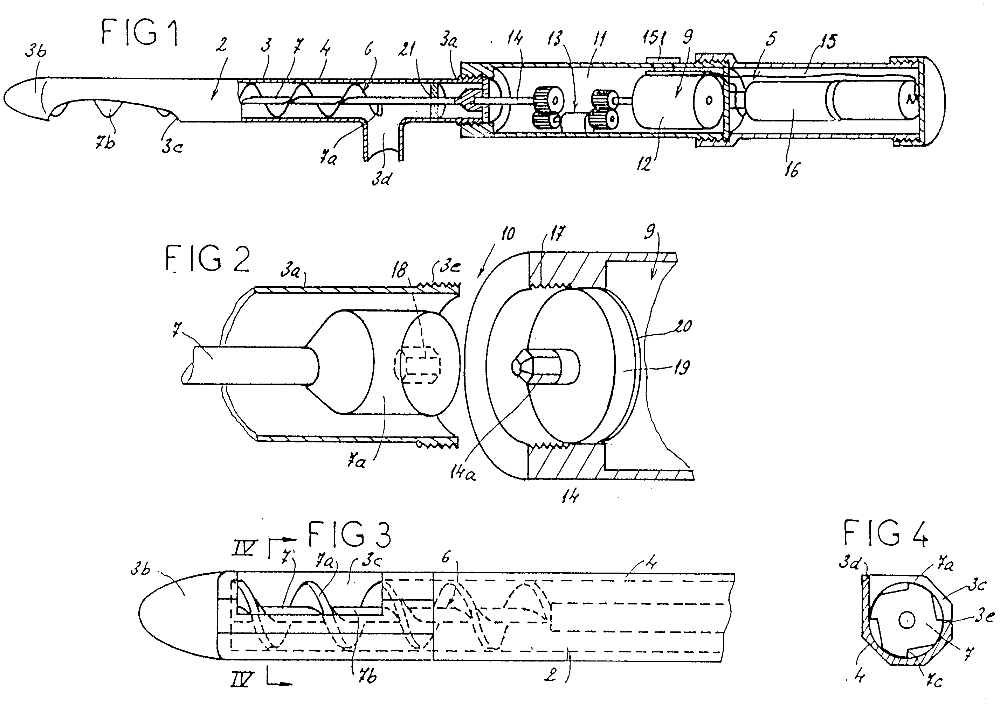

Conformément à la figure 1, un instrument selon l'invention comprend, d'un côté un outil 2 de fragmentation du matériau vivant, travaillant à sec, et éventuellement à usage unique, c'est-à-dire jetable, et de l'autre côté le moyen d'actionnement 5 dudit outil, raccordé à ce dernier selon les dispositions représentées à la figure 2, et décrites ci-après.

L'outil de fragmentation 2 comprend un manchon droit 3 d'insertion dans l'organe traité (en particulier dans l'oeil), allongé d'une extrémité distale 3a destinée à rester à l'extérieur de l'organe traité, à une extrémité proximale 3b de forme convexe, plus précisément ogivale pour protéger et éloigner les tissus non concernés, et destinée à venir à l'intérieur de l'organe traité, en relation avec le matériau vivant à fragmenter et extraire. Ce manchon comporte une fenêtre 3c antérieure, située en totalité dans la paroi latérale 4 dudit manchon, du côté de son extrémité proximale, et mettant en communication l'intérieur et l'extérieur dudit manchon. Ce dernier détermine, de manière adjacente à son extrémité distale 3a, un conduit d'évacuation 3d, perpendiculaire à la direction du manchon 3, et pouvant se limiter à une simple fenêtre.

A l'intérieur du manchon 3, est disposé un organe rotatif 6 de découpe et évacuation longitudinale du matériau vivant. Cet organe rotatif 6 consiste en une vis d'évacuation 7 du matériau vivant découpé, vers l'extrémité distale 3a de l'outil 2 de fragmentation, cette vis 7 étant disposée coaxialement avec le manchon 3.

La partie antérieure 7b de la vis 7 d'évacuation est disposée en vis-à-vis de la fenêtre antérieure 3c du manchon 3. Comme montré plus particulièrement par la figure 4, le filet 7a de la vis 7 présente éventuellement une dépouille 7c de coupe, selon toute sa longueur et en particulier dans sa partie antérieure 7b, ceci pour constituer directement un moyen rotatif de coupe du matériau vivant. Selon la figure 1, la vis 7 constitue donc à la fois un organe rotatif de découpe, grace à son filet de coupe 7a, et un organe d'évacuation longitudinale du matériau vivant découpé, à la manière d'une vis d'Archimède.

De manière non représentée, la fenêtre antérieure 3c présente éventuellement une section asymétrique, de section relativement étroite au voisinage de son extrémité proximale, et de section relativement large à son extrémité distale. Comme montré en particulier par la figure 4, les bords 3d et 3e de la fenêtre 3c sont évasés et arrondis. Et comme l'extrémité proximale 3b du manchon 3 est complètement fermée, la fenêtre antérieure 3c est située complètement dans la paroi latérale 4 du manchon 3.

Le moyen d'actionnement 5 de l'outil 2 de fragmentation comprend un moyen mécanique 9 d'entraînement en rotation de l'organe rotatif 6 de découpe et d'évacuation longitudinale du matériau vivant. Plus précisément, le moyen d'actionnement 5 de forme généralement cylindrique comporte, d'un côté un premier compartiment 11 rassemblant un moteur électrique 12, un réducteur 13, un arbre de sortie 14, un interrupteur 151 commandant le moteur électrique 12, éventuellement un variateur de vitesse et une commande d'inversion du sens de rotation, et un deuxième compartiment 15 contenant des piles ou accumulateurs électriques 16, alimentant le moteur 12. Le raccord 10 représenté à la figure 2 permet, d'une part de fixer l'extrémité distale 3a de l'outil 2 sur la partie antérieure du premier compartiment 11, grâce à un filetage 3e du manchon 3 coopérant avec un taraudage 17 prévu sur la paroi cylindrique du compartiment 11, et d'autre part d'accoupler en rotation l'arbre de sortie 14 avec la vis 7, grâce à une extrémité mâle 14a de section hexagonale de l'arbre 14 susceptible de pénétrer dans un organe femelle 18, de forme et section adaptées, prévu en creux sur l'extrémité distale 7a de la vis 7. L'arbre de sortie 14 est tourillonné sur une rondelle métallique 20 fermant le compartiment 11, l'étanchéité de ce dernier étant assurée par un joint 19. Du côté du manchon 3, l'extrémité distale 7a de la vis est elle-même tourillonnée sur une entretoise 21 fermant le manchon 3 de manière étanche, à l'opposé de la fenêtre 3c.

Le manchon représenté aux figures 3 et 4 ne diffère de celui représentéà la figure 1 que par la section de forme généralement octogonale dudit manchon, et la fenêtre 3c de forme rectangulaire.

A titre de variante, et de manière non représenté l'outil 2 et le moyen d'actionnement 5 peuvent être séparés et à distance l'un de l'autre, en étant reliés par une transmission en rotation par flexible. Une telle disposition simplifie en particulier la stérilisation de l'instrument, cette dernière étant strictement limitée à l'outil.

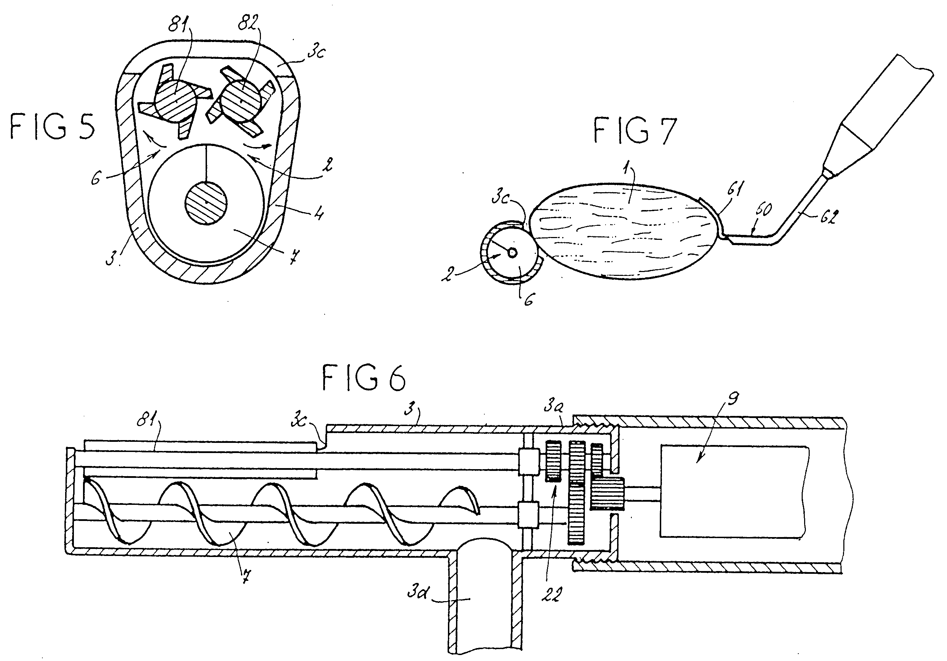

Conformément aux figures 5 et 6, où les fonctions de coupe et évacuation longitudinale son séparées, l'organe rotatif 6 de découpe est distinct de la vis d'évacuation 7, et comprend deux fraises 81 et 82, disposées de manière adjacente, toutes les deux en vis-à-vis de la fenêtre antérieure 3c, tournant en sens opposé pour amener le matériau vivant à l'intérieur du manchon 3. La vis d'évacuation 7 est disposée dans le manchon 3, de manière adjacente aux fraises 81 et 82, du côté de ces dernières opposé à la fenêtre antérieure 3c.

Comme représenté à la figure 6, à partir du moteur électrique 9, des moyens 22 du type pignons permettent d'entraîner en rotation, à la fois la vis 7, et les deux fraises 81 et 82, en sens inverse l'une par rapport à l'autre.

L'outil de fractionnement 2 peut être construit pour évacuer le matériau vivant fragmenté par la sortie 3d. Mais la longueur du manchon 3 d'insertion, par rapport à la vis d'évacuation 7, est avantageusement adaptée à un stockage du matériau vivant découpé et évacué, entre la vis 7 et manchon 3 sur toute sa longueur ; ceci permet de jeter l'outil de fragmentation 2, avec le matériau vivant fragmenté stocké dans le manchon 3.

Conformément à la figure 7, on a représenté le mode d'utilisation de l'instrument précédemment décrit, pour une intervention chirurgicale effectuée, sur l'oeil, limité dans la représentation de la figure 7 au cristallin 1. Ce dernier est attaqué d'un côté, tangentiellement, par l'outil 2, introduit par une première incision dans la cornée, dont la fenêtre 3c est ouverte sur le cristallin, et est maintenu de l'autre côté par un instrument du type spatule 60, également introduit par une deuxième incision dans la cornée. Cet instrument comprend une partie plate 61 de profil adapté à un contact non blessant avec le cristallin, et une partie creuse 62 amenant un matériau visqueux tel que collagène en remplacement du cristallin. Plus précisément, par rotation de la vis 7 dans la fenêtre antérieure 3c, on découpe et fragmente le noyau dur, dont les morceaux sont évacués par la même vis 7. Le filet 7a de la vis 7 a non seulement pour fonction de découper ou entailler le cristallin 27, mais également d'obturer et réouvrir périodiquement la fenêtre antérieure 3c, à la manière d'une bouche qui s'ouvre et se ferme successivement pour avaler les morceaux fragmentés.

## Revendications

**1)** Instrument chirurgical pour la fragmentation in situ d'un matériau vivant dans un organe (1) ou une partie d'un corps humain ou animal, et l'extraction dudit matériau dudit organe, ledit instrument comprenant, d'une part un outil de fragmentation (2) comprenant un manchon (3) d'insertion dans ledit organe, allongé d'une extrémité distale (3a) destinéeà rester à l'extérieur dudit organe, à une extrémité distale (3a) destinée à rester à l'extérieur dudit organe, à une extrémité proximale (3b) destinée à venir à l'intérieur dudit organe, en relation avec le matériau vivant, et comportant une fenêtre (3c) antérieure située au moins pour partie dans la paroi latérale (4) dudit manchon, du côté de son extrémité proximale, mettant en communication l'intérieure et l'extérieur dudit manchon, et d'autre part un moyen d'actionnement (5) de l'outil au contact direct du matériau vivant, à fragmenter et extraire, caractérisé en ce que l'outil est un outil de travail à sec du matériau vivant et comprend a l'intérieur du manchon un organe rotatif (6) de découpe et évacuation longitudinale du matériau vivant, associant, d'une part au moyen rotatif de coupe (7a, 81, 82), en vis-à-vis de la fenêtre antérieure (3c), susceptible de découper le matériau vivant au travers de ladite fenêtre, et d'autre part une vis d'évacuation (7) du matériau vivant découpé, vers l'extrémité distale (3a) de l'outil (2), et le moyen d'actionnement (5) d'entraînement en rotation de l'organe rotatif (6) de découpe et évacuation longitudinale du matériau vivant.

**2)** Instrument selon la revendication 1, caractérisé en ce que la partie antérieure (7b) de la vis (7) d'évacuation est disposée en vis-à-vis de la fenêtre antérieure (3c), et le filet (7a) de ladite vis présente une dépouille (7c) de coupe, au moins dans sa partie antérieure (7b), pour constituer directement le moyen rotatif de coupe du matériau vivant.

**3)** Instrument selon la revendication 1, caractérisé en ce qu'au moins une fraise (81, 82) est disposée vis-à-vis de la fenêtre antérieure (3c), et la vis d'évacuation (7) est disposée dans le manchon (3) d'insertion, de manière adjacente à la fraise, du côté de cette dernière opposé à la fenêtre antérieure (3c).

**4)** Instrument selon la revendication 3, caractérisé en ce que deux fraises (81, 82), tournant en sens opposé pour amener le matériau vivant à l'intérieur du manchon (3), sont disposées en vis-à-vis de la fenêtre antérieure (3c).

**5)** Instrument selon la revendication 1, caractérisé en ce que la fenêtre antérieure (3c) présente une section, notamment asymétrique proximale et de section relativement large à son extrémité distale.

**6)** Instrument selon la revendication 1, caractérisé en ce que les bords (3d, 3e) de la fenêtre (3c) antérieure sont évasés et arrondis.

**7)** Instrument selon la revendication 1, caractérisé en ce que l'extrémité proximale (3b) du manchon (3) est fermée, moyennant quoi la fenêtre antérieure (3c) est située complètement dans la paroi (4) latérale du manchon (3).

**8)** Instrument selon la revendication 1, caractérisé en ce qu'il comprend d'un côté le moyen d'actionnement (5) et de l'autre côté l'outil de fragmentation (2) à sec, à usage unique, et à cette fin l'outil de travail à sec et/ou le moyen d'actionnement comprennent un raccord (10) permettant notamment d'accoupler en rotation le moyen mécanique (9) d'entraînement en rotation avec l'organe rotatif (6) de découpe et évacuation longitudinale du matériau vivant.

**9)** Instrument selon la revendication 1, caractérisé en ce que la longueur du manchon (3) d'insertion par rapport à la vis d'évacuation (7) est adaptée à un stockage du matériau vivant découpé et évacué.

**10)** Instrument selon l'une quelconque des revendications 1 à 9, caractérisé en ce qu'il est un instrument de phaco-fragmentation du cristallin de l'oeil.
